# EUROPEAN PATENT SPECIFICATION

(11) **EP 4 518 914 B1**
(45) Date of publication and mention of the grant of the patent: **25.11.2026**
(21) Application number: 22729050.9
(22) Date of filing: 06.05.2022
(51) Int. Cl.: A61L 9/02, A61L 9/03

(54) **APPARATUS FOR DISPENSING VOLATILE SUBSTANCES AND METHOD FOR PRODUCING THE SAME**
VORRICHTUNG ZUR ABGABE VON FLÜCHTIGEN SUBSTANZEN UND VERFAHREN ZU IHRER HERSTELLUNG
APPAREIL DE DISTRIBUTION DE SUBSTANCES VOLATILES ET SON PROCÉDÉ DE PRODUCTION

(43) Date of publication of application: 12.03.2025
(73) Proprietor: CTR, Lda, 2135-301 Samora Correia (PT)
(72) Inventor: HISHIKI, Teruo, Tokyo, 166--0012 (JP); AUGIER, Harold, Zhongshan City, Guangdong 528458 (CN)
(74) Representative: Liebl, Thomas
(86) International application number: PCT/EP2022/062364
(87) International publication number: WO 2023/213414

(56) References cited:
- EP-B1- 1 701 614
- WO-A1-2021/146128
- ES-A1- 2 734 913
- ES-B1- 2 178 906
- US-A1- 2021 289 588

## Description

The invention relates to a device for dispensing, in particular for evaporating, volatile substances, in particular fragrances and/or active substances, according to claim 1. Furthermore, the invention relates to a method for manufacturing such a device, in particular for manufacturing a heating device of such a device, according to claim 15.

A known device for dispensing, in particular for evaporating volatile substances, is known from WO 2017/2157287 A1 and from US 5,903,710 A and has a container for the substance to be dispensed and a capillary element which is arranged at least partially in the container and which is in contact with the substance to be dispensed and conveys it by means of capillary action to at least one substance dispensing area. Furthermore, an electrical heating device is provided which has an electrical heating resistor in the region of the capillary element which is in contact with the substance dispensing area. In addition, a container holder is provided to which the container can be connected and which has an electrical connection element by means of which the electrical heating device can be supplied with electrical energy. The electrical connection element is specifically designed here as a plug component with projecting plug contacts that can be plugged into an electrical socket for electrical power supply. ES2734913 A1 and WO 2021/146128 A) relate to devices for dispensing fragrances through a wick which is heated by multiple heater elements.

In the aforementioned prior art, the electrical heating resistor is represented as a relatively large areal element, which can also be formed, among other things, by spraying or printing and drying an electrically conductive ink or paint that has a resistance function. As a disadvantage, uneven temperature distributions can occur here due to the large areal area, in particular with temperature hot spots, which can lead to damage in the capillary element, especially at initial, high switch-on temperatures. In particular, a free capillary element end can dry out and dehumidify considerably as a result of temperature hot spots, whereby the capillary structure can be damaged by sticking and/or caking together to such an extent that the delivery rate for the substance to be dispensed is considerably reduced and, in extreme cases, no more substance evaporates and a partially still filled container has to be replaced. Moreover, the production of a relatively large heating element area using an electrically conductive ink or paint is difficult in terms of production with regard to uniform distribution and drying and can lead to uneven layer thicknesses, which in turn promote uneven temperature distribution in conjunction with temperature hot spots. In addition, the prior art does not provide any specific information on how to implement the simplest possible and most functionally reliable contacting for supplying energy to the electrical, planar heating resistor there.

It is the object of the invention to create a device for dispensing, in particular for evaporating, volatile substances, in particular fragrances and/or active ingredients, with regard to its electrical heating device in such a way that gentle and long-lasting operation of the capillary element with a high degree of evaporation is possible with a simple and inexpensive structure as well as simple manufacture. A further object of the invention is to propose a manufacturing method suitable for this purpose.

The object of the invention is solved with respect to the device with the features of independent claim 1. Advantageous embodiments thereof are the subject of the subclaims referring back thereto.

According to claim 1, there is provided a device for dispensing volatile substances comprising:
- a container for the substance to be dispensed,
- at least one capillary element which is arranged at least partially in the container and which is in contact with the substance to be dispensed and conveys it by means of capillary action to at least one substance dispensing area,
- an electrical heating device with at least one electrical heating resistor arranged in the region of the capillary element,
- an electrical connection element by means of which the electrical heating device can be supplied with electrical energy.

According to the invention, it is further provided:
- that the heating resistor is formed by at least two element rows spaced apart from one another, each element row having at least one areal heating element, preferably at least one PTC areal heating element,
- that the areal heating elements are applied as film layer resistors to an electrically non-conductive base carrier layer,
- that an electrically conductive film layer conductor grid is applied to the base carrier layer for supplying electrical energy to the areal heating elements.

It has been shown that with the claimed division of the electrical heating resistor into spaced element rows and individual areal heating elements, a uniform temperature distribution in the area of the capillary element can be achieved, in particular without harmful temperature hot spots. This avoids any harmful influence on the capillary element and can ensure uniform, safe operation of the device.

The areal heating elements are preferably PTC areal heating elements, i.e. heating elements whose material has a positive temperature coefficient, which means that their electrical resistance also increases with rising temperature. Specifically, this means that after the device is switched on, the PTC function of the PTC areal heating elements does not yet take effect immediately, so that a comparatively high temperature of, for example, about 70° C to 110° C is initially generated with the heating device, depending on the choice of materials used and the design structure. For this purpose, an electrical power of 5 watts may be required as an example. As the temperature rises, this is reduced again by the use of the PTC function, for example to operating temperatures of approx. 60° C, whereby the required electrical power then also decreases, for example to 1 watt.

Further, according to a particularly preferred specific embodiment of the device, it is provided:
- that a first main line and a second main line of the film layer conductor grid are arranged on opposite sides of the at least two rows of elements and can each be connected to poles of different voltage of the electrical connection element,
- that, starting from the first main line, a respective stub line runs along each row of elements and makes electrical contact with the at least one areal heating element of each row of elements,
- that, starting from the second, opposite main line, a respective stub line runs along each row of elements and makes electrical contact with the at least one areal heating element of each row of elements, and
- in that the stub lines of the first main line and the stub lines of the second main line each have a stub line distance between assigned areal heating elements of an element row.

In addition to being easy to manufacture, such a design also results in a particularly advantageous compact arrangement with functionally reliable contacting and power supply.

The power supply, and in particular a DC power supply, depends on the material used for the areal heating elements and can be, for example, the residential voltage or a reduced voltage, e.g. in the range of 5 to 12V, for e.g. PTC ink materials.

Moreover, the above advantages can be achieved in particular if, according to a particularly preferred embodiment, at least two areal heating elements spaced apart from each other with intermediate distances are arranged in an element row and/or an equal number of areal heating elements is provided in each element row.

The production, in particular by a printing or spraying process, with electrically conductive ink or electrically conductive paint can be carried out more easily, more reliably and more uniformly with an arrangement of relatively small areal heating elements compared with large-area applications. A particular advantage also lies in the provision of an electrically conductive film layer conductor grid, which can also be produced simply and inexpensively and results in a functionally reliable electrical bond between the electrical conductors and the areal heating elements. Electrical contacting is thus achieved here by means of a relatively large-area contacting composite, i.e. by means of intensive contact without weak contact points and tolerances.

In principle, there are various options as to how or in which order the areal heating elements and the film layer conductor grid can be applied to the base carrier layer: According to a first variant, the areal heating elements can be applied to the base carrier layer before the film layer conductor grid and thus lie below the stub liness subsequently applied to the base carrier layer. Alternatively, the film layer conductor grid can be applied to the base carrier layer before the areal heating elements, and then the areal heating elements can be applied to the base carrier layer so that the areal heating elements lie on top of the stub lines.

According to a particularly preferred embodiment, the areal heating elements are formed by an electrically conductive ink or paint having a resistance function, preferably a PTC resistance function, which is applied to the base carrier layer as a film layer, preferably as a thin film layer, preferably printed and dried.

Furthermore, the electrically conductive film layer conductor grid can also be formed by an electrically conductive ink or ink having a current-carrying function, preferably having silver, which is applied to the base carrier layer as a film layer, preferably as a thin film layer, preferably printed and dried.

In a particularly preferred embodiment, the areal heating elements each have the same design and/or the areal heating elements have a rectangular shape, in particular a square shape, whereby it is preferably provided that the areal heating elements have edge lengths of 2 mm to 8 mm. Rectangular areal heating elements can be easily calculated and designed and therefore ensure high quality and precision. The specified edge lengths have also proved to be particularly advantageous limits in conjunction with specific arrangements, with which, on the one hand, a desired heating output can be achieved and, on the other hand, undesirable hot spots can be reliably avoided.

According to a further particularly preferred concrete embodiment, two to six, preferably two to four, element rows, each with two to five, preferably three or four, areal heating elements, are arranged in parallel, preferably with a respective equal row spacing between mutually opposite areal heating elements of adjacent element rows and/or with a respective equal intermediate spacing between adjacent areal heating elements of an element row. This makes it possible to generate uniform areal patterns which, on the one hand, can be produced simply and inexpensively and, on the other hand, ensure a favourable, uniform temperature distribution in the region of the at least one capillary element.

In order to achieve particularly suitable results with a very compact structure, the row spacing defined by the distance between two adjacent areal heating elements of adjacent element rows is preferably 1 mm to 5 mm. Alternatively or additionally, the distance between two adjacent areal heating elements of an element row is 0.5 mm to 5 mm.

For the required, functionally reliable contacting with a compact structure, the first main line and the second main line of the film layer conductor grid are preferably each aligned laterally and essentially perpendicular to the element rows and have a main line width of 1 mm to 5 mm, preferably of 1 mm to 3 mm. Alternatively or additionally, for the same reason, the stub lines can be aligned parallel and associated with the element rows, and preferably with a comparatively smaller stub line width of 0.3 mm to 1 mm.

Particularly preferably, the two stub lines of an element row have a stub line spacing of at least 1 mm or 1 mm or greater, with the stub line spacing between the two stub lines always being selected so that the two stub lines run within the area of the associated areal heating elements. This means that in each case areas of the areal heating elements project beyond the stub lines, so that the stub lines have a secure contact bond with the associated areal heating elements over their entire width even in the event of tolerances in manufacture.

A particularly compact and material-saving design of the heating device, while still providing the desired heating power in a functionally reliable manner, can be achieved if the layer thickness of the areal heating elements formed by film layer resistors and/or of the film layer conductor grid is 0.5 µm to 30 µm, preferably 5 µm to 25 µm, most preferably 10 µm to 20 µm.

With the dimensions and arrangements explained above, the evenly distributed temperatures suitable for operation of the device can be realized particularly well, especially when using commercially available inks or paints for the production of the areal heating elements and the film layer conductor grid.

For example, materials that available on the market as inks can be used for the production of both the areal heating elements and the film layer conductor grid, which are specifically applied to the base carrier layer with a layer or film thickness of approx. 15 µm. For example, Loctite^{®} ECI 8001 or Loctite^{®} 8120 can be used to produce the areal heating elements and Loctite^{®} ECI 1010 or Loctite^{®} ECI 4002 can be used to produce the film layer conductor grid. More generally, the base material for the areal heating elements is preferably a PET material or a polyamide material, the latter being more suitable for higher temperatures.

In the following, two particularly preferred but different and alternative devices are explained by way of example only:
In the first embodiment, the base carrier layer is formed by the container or is a component of the container. Further, a container holder is provided to which the receptacle is detachably connectable, the container holder further comprising the electrical connection element to which the receptacle is electrically connectable. Such a structure is simple to manufacture and takes up relatively little space. To establish the electrical connection with the areal heating elements, an electrical contact device, preferably a contact device with plug segments, is provided on the main lines, by means of which the two main lines for supplying electrical power to the areal heating elements can be connected directly or indirectly to the electrical connection element.

Further, in this first embodiment, it is provided that the container holder is configured as a plug component, wherein the electrical connection element has protruding plug contacts that can be plugged into an electrical socket for supplying electrical power to the areal heating elements and for holding the plug component as well as the container connected to the plug component.

In this first embodiment, the base carrier layer can be directly a non-conductive outer side of a container wall part or a non-conductive intermediate layer connected to a container wall part, with plastic material preferably being used here. In a heat transfer region, a capillary element region to be heated as a substance dispensing area is to be adjacent to the base carrier layer with the areal heating elements, if necessary with the interposition of an areal heat transfer element, in particular in areal contact.

Particularly suitable for this purpose is a capillary element which is designed as a U-shaped, flat wick which is in contact with the substance to be dispensed by means of both U-legs in the container, the U-base part forming the substance dispensing area and this resting against a heat transfer area. For the release of the substance to the environment, a substance outlet opening which can be opened for the operating state is also preferably arranged in the otherwise closed container.

In an alternative, second embodiment, a container holder is provided to which the container can be detachably connected, the container holder being formed by a housing component in which the container is at least partially accommodated. In addition, the base carrier layer is here, together with the areal heating elements, a component of the housing component and is arranged in the interior of the housing component in the region of the inserted container, the housing component having the electrical connection element to which the container can be electrically connected.

In this second embodiment, it is preferably provided that the base carrier layer with the areal heating elements is arranged in the region of a substance dispensing area of a capillary element to be heated, most preferably associated with a free wick end of a wick projecting from the container as a capillary element.

Furthermore, in this second embodiment, it is preferably provided that the housing component is designed as a plug component, the electrical connection element having projecting plug contacts which can be plugged into an electrical socket for supplying electrical power to the areal heating elements and for holding the plug component and the container connected to the plug component. In this embodiment, the base carrier layer with the areal heating elements is thus an integral part of the housing component and not of the container. The areal heating elements can also be attached, for example, to a ring heating element as the base carrier layer, which surrounds a free wick end projecting from the container.

The object of the invention is solved with respect to the method with the features of independent claim 22. Advantageous embodiments thereof are the subject of the subclaims referring back thereto.

The advantages resulting here are identical to those of the device, so that to avoid repetition, reference is made to the explanations previously given concerning the device.

The invention is further explained by way of example with the help of the following figures:
- Fig. 1: an electrical, areal heating resistor with PTC areal heating elements of a first embodiment;
- Fig. 2: an electrical, areal heating resistor with PTC areal heating elements of a second embodiment;
- Fig. 3: a schematic illustration of a container holder and a container;
- Fig. 4: a perspective view of a modified container obliquely from above with a capillary element and a heating resistor;
- Fig. 5: a representation of the container according to Fig. 4, viewed obliquely from below;
- Fig. 6: an exploded view of the container according to Fig. 4;
- Fig. 7: an alternative embodiment to the illustrations according to Figs. 3 to 6 with a container holder as a housing component;
- Fig. 8: a partial section along line A-A of Fig. 7.

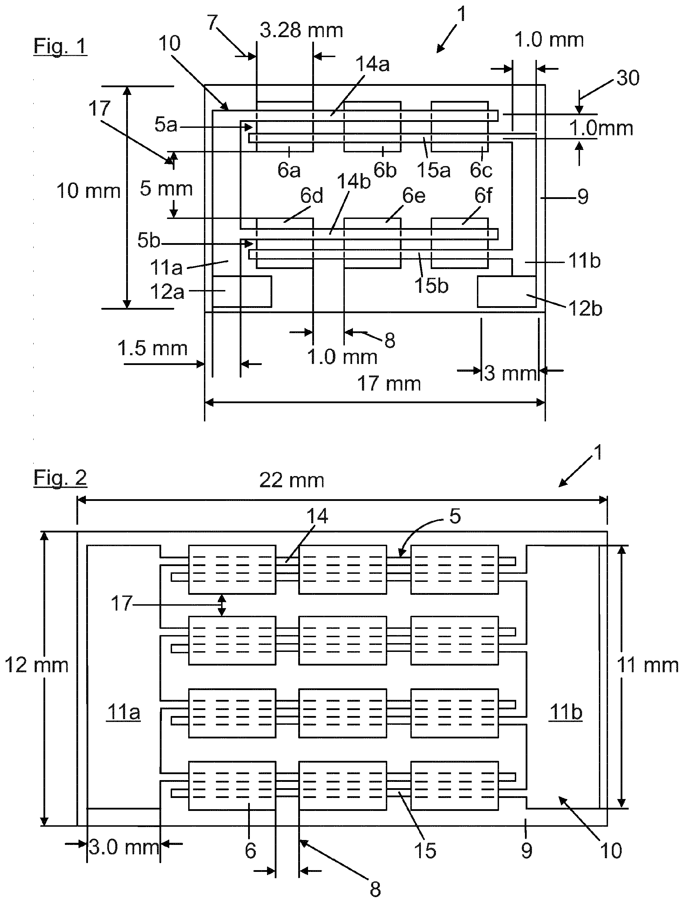

Figs. 1 and 2 show a plan view of two different embodiments of an exemplary electrical and areal heating resistor of an electrical heating device, which are used in a device for dispensing volatile substances, in particular fragrances and/or active ingredients, which has a container 3 for the substance to be dispensed.

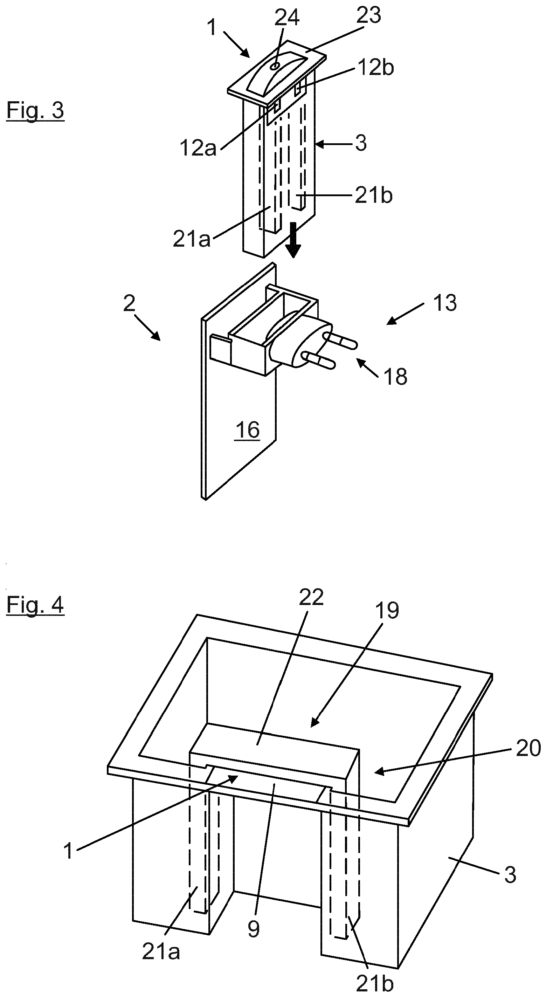

In a first embodiment of such a device, also referred to as a dispenser, shown in Figs. 3 to 6 in this respect, a container holder is designed as a plug component 2, the heating resistor 1 being a component of the container 3 here by way of example, or in other words being integrated into the container 3.

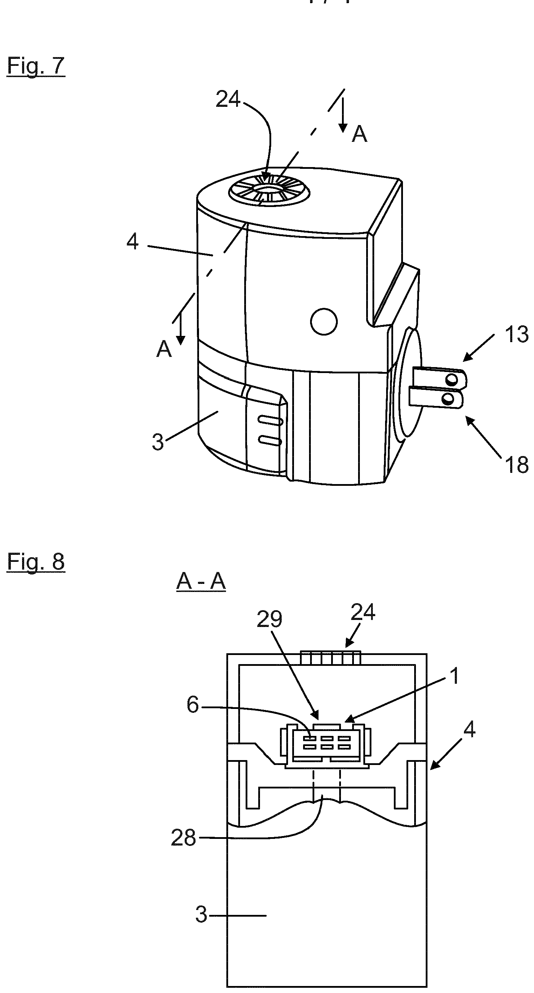

In an alternative embodiment of such a dispensing device, as shown in Figs. 7 and 8, the container holder is formed as a housing component 4 into which the container 3 is inserted, the heating resistor 1 not being a component of the container 3 here, but being a component of the housing component 4.

Referring to Fig. 1, the areal heating resistor 1 has a plurality of element rows 5, here exemplarily two, spaced apart from each other, hereinafter referred to as element rows 5a and 5b. These element rows 5a, 5b each have, here exemplarily, a plurality of, for example three, spaced-apart areal heating elements 6, which are hereinafter referred to as areal heating elements 6a, 6b, 6c and 6d, 6e, 6f and are which are here further exemplarily PTC areal heating elements. These PTC areal heating elements 6a to 6f preferably each have the same geometry or shape, here exemplarily each having the same rectangular or square shape. The square-shaped PTC areal heating elements 6a to 6f can, for example, have an edge length 7 of 3.28 mm and, for example, the intermediate distance 8 between the individual PTC areal heating elements 6a to 6f within an element row 5a, 5b is approximately 1 mm. The row spacing 17 between the oppositely assigned PTC areal heating elements 6a to 6f of the element rows 5a, 5b is approximately 5 mm.

It has been shown that, in general, distances (intermediate distances, row distances) greater than 5 mm increase the risk of damaging temperature hot spots and, accordingly, smaller distances (intermediate distances, row distances) of approximately 1 mm are particularly suitable for forming the heating resistor 1.

The PTC areal heating elements 6a to 6f are preferably formed by film layer resistors, which are preferably produced by an electrically conductive ink or paint having a resistance function, preferably a PTC resistance function, and are printed and dried as a film layer on a base carrier layer 9. For example, such an electrically conductive ink or paint is available on the market under the designation Loctite^{®} ECI 8001 or Loctite^{®} ECI 8120, which has the advantage that it can be applied to the base carrier layer 9 with a very thin film layer thickness, e.g. with a film layer thickness of 15 µm. The PTC function advantageously leads here with a relatively high switch-on temperature of, for example, about 70° C to 110° C to a rapid substance release after switching on, the temperature then subsequently decreasing to a desired operating temperature, for example to about 60° C, due to the effectiveness of the PTC function and an associated reduction in the electrical power consumption, for example from about 5 watts to about 1 watt of operating power.

For the electrical power supply of the PTC areal heating elements 6a to 6f, an electrically conductive film layer conductor grid 10 is applied to the base carrier layer 9, e.g. also with a film layer thickness of 15 µm. Similar to the PTC areal heating elements 6a to 6f, this film layer conductor grid 10 is printed onto the base carrier layer 9 by means of an ink or paint and dried, whereby here the ink or paint has an electrically conductive function, which is preferably due to silver or copper particles. For example, such materials are available on the market under the designation Loctite^{®} ECI 1010 or Loctite^{®} ECI 4002.

The film layer conductor grid 10 has a first main line 11a and a second main line 11b which, in the example shown here, each run opposite one another laterally and transversely to the two rows of elements 5a, 5b and which can each be connected to poles of different voltage of an electrical connection element, e.g. can be connected to poles of different voltage of an electrical connection element of a container holder, as will be explained in more detail below with reference to the individual embodiment examples:
For example, if the heating resistor 1 is arranged on the container 3 in an embodiment according to Figures 3 to 6, an electrical contact device with plug elements 12a, 12b can also be provided on the first main line 11a and on the second main line 11b, respectively, with which an electrical contact can be made to the electrical connection element 13 thereof when the container 3 is connected to the plug component 2 acting as a container holder.

Here, for example, the first main line 11a and the second main line 11b have a width of approx. 1 mm. Starting from the first main line 11a, two stub lines 14a, 14b run in the example shown here with an electrical contact and interconnection via the PTC areal heating elements 6a, 6b, 6c and via the PTC areal heating elements 6d, 6e, 6f, respectively.

Starting from the second main line 11b, two stub lines 15a, 15b also run with an electrical contact and interconnection via the PTC areal heating elements 6a, 6b, 6c and via the PTC areal heating elements 6d, 6e, 6f of each element row 5a and 5b, respectively. The stub lines 14a, 15a and the stub lines 14b, 15b of each element row 5a and 5b, for example, each have a stub line spacing 30 of approx. 1 mm and thus run well within the area of the PTC areal heating elements 6a to 6f, so that a reliable contact overlap is ensured even with manufacturing tolerances. The stub lines 14a, 14b and 15a, 15b have a smaller stub line width than the main lines 11a, 11b, for example approx. 0.3 mm.

The second embodiment shown in Fig. 2 of a flat heating resistor 1 which can be used in a dispensing device is constructed similarly to the heating resistor 1 in Fig. 1, so that only the differences will be explained below, using largely the same reference signs:
A significant difference here is that the film layer conductor grid 10 was printed onto the base carrier layer 9 before the PTC areal heating elements 6, and then the PTC areal heating elements 6 were produced in a further printing process, as a result of which the film layer conductor grid 10 is located below the PTC areal heating elements 6, with a very good and reliable electrical contact bond, as in Fig. 1.

Instead of the merely exemplary two element rows 5a, 5b according to Fig. 1, four parallel element rows 5 are provided in the embodiment according to Fig. 2 - again merely exemplary - which here likewise merely exemplarily each have three PTC areal heating elements 6. In contrast to Fig. 1, the row spacing 17 between the two element rows 5a, 5b in the embodiment according to Fig. 2 is somewhat smaller here merely to illustrate the basic possibility of varying the spacing, but could also be the same or somewhat larger. The PTC areal heating elements 6 each have a rectangular shape here merely by way of example, with the element length, viewed in the direction of the element rows, being, for example, approximately 3 mm and/or with the element width, viewed transversely to the element row, being, for example, approximately 2 mm.

With both the embodiment of the heating resistor 1 according to Fig. 1 and Fig. 2, the required small dimensions of the entire heating resistor arrangement of about 10 to 12 mm with suitable heating power for accommodation in a dispenser can be realized.

Fig. 3 schematically shows the first embodiment of the device as a dispenser for volatile substances, in particular fragrances and/or active ingredients. In this embodiment, the container holder for the container 3 is designed as a plug component 2, in which an electrical connection element 13 is mounted on a cover plate 16. The cover plate 16 may have design elements (not shown) in the visible area, wherein the electrical connection element 13 also has plug contacts 18 which can be plugged into an electrical socket (not shown) for supplying electrical power to the PTC areal heating elements 6 and for holding the plug component 2 with the container 3. The substance to be dispensed, in particular in the form of a liquid, is accommodated in the container 3, with a capillary element 19, which is designed here merely by way of example as a U-shaped flat wick 20 with U-legs 21a, 21b, being immersed so that its upper U-base region forms a substance dispensing region 22 which is to be heated by means of the heating resistor 1.

In Fig. 3, the substance dispensing area 22 and also the heating resistor 1 are exemplarily covered with a cover hood 23, which has an outlet opening 24 for the substance to be dispensed.

Fig. 4 shows a container 3 with a slightly modified design compared to Fig. 3 in the open top state, in which the substance to be dispensed is accommodated during operation. Here, too, a U-shaped flat wick 20 is shown as capillary element 19, which is immersed with its U-legs 21a, 21b downward into the substance to be dispensed. The U-base as substance dispensing area 22 rests here from above on an electrically non-conductive base carrier layer 9 of an upper shoulder or projection of the container 3. In the embodiment shown in Fig. 4, the flat heating resistor 1 with the PTC areal heating elements 6 is arranged on the base carrier layer 9 under the substance delivery area 22 (not visible), whereby the substance delivery area 22 here rests directly on and against the electrical heating resistor 1.

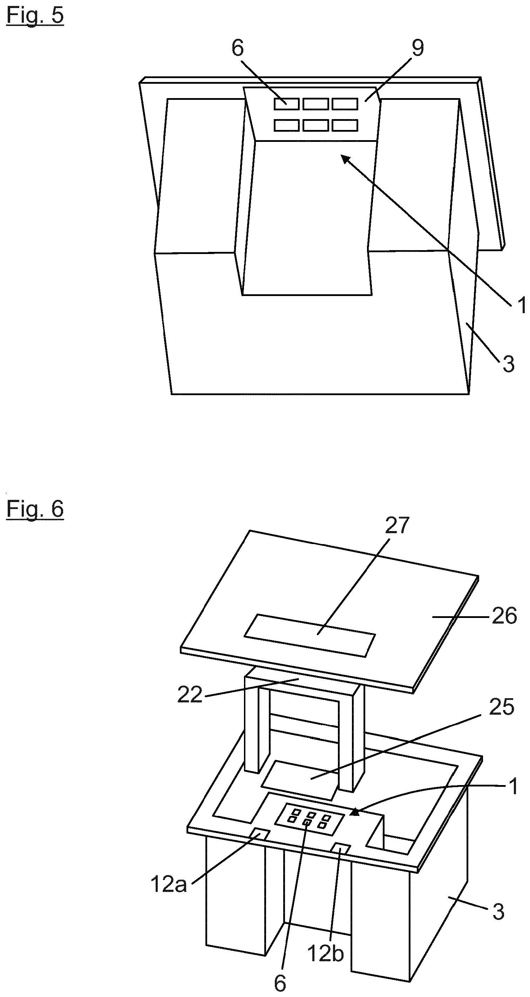

Fig. 5 shows a modified embodiment of the container 3 shown in Fig. 4 with regard to the arrangement of the heating resistor 1. Here, the base carrier layer 9 with the PTC areal heating elements 6 is attached under the upper wall shoulder on which the substance dispensing area 22 of the flat wick 20 rests. This means that heat is transferred here by conduction through the upper wall shoulder to the substance dispensing area 22. The base carrier layer 9 can therefore also be an electrically non-conductive wall area of the container 3 on which the heating resistor 1 is printed.

Fig. 6 again shows schematically in an exploded view a modified embodiment of the container 3. Here, too, the heating resistor 1 with the PTC areal heating elements 6 is arranged on an upper wall shoulder of the container 3 and is equipped with plug-in elements 12a, 12b for connection to electrical connection elements 17 of a plug component 2 acting as a container holder. A heat-conducting, preferably thin plate 25, for example an aluminium plate, is arranged here on the heating resistor 1 and under the substance dispensing area 22 for uniform heat distribution to the substance dispensing area 22. The container 3 is here only exemplarily covered from above with a tightly attached, flat cover element 26, which has a dispensing opening 27 above the substance dispensing area 22. The dispensing opening 27, or as shown in Fig. 3 as a smaller outlet opening 24, is still closed in the sales packaging of the container and can be opened for operation before the container 3 is inserted into the plug component 2, in particular by tearing open a film seal.

In an alternative embodiment of the dispenser device shown in Fig. 7, the container holder is designed as a housing component 4 and, in addition, here as a plug component by way of example, which has an electrical connection element 13 with plug contacts 18 which can be plugged into an electrical socket (not shown) for an electrical power supply to the PTC areal heating elements 6 and for holding the housing component 4 and the container 3 which is also partially inserted therein.

From the sectional view according to Fig. 8, it is also evident that the container 3 is partially inserted into the housing component 4 from below and connected to it in a manner known per se, with a wick 28 in the form of a capillary element projecting upwards from the container 3 with its free wick end 29. In the region of the free wick end 29, the heating resistor 1 with the PTC areal heating elements 6 is fixedly arranged here in a schematic representation as a component of the housing component 4. Depending on the local conditions, the base carrier layer 9 with the PTC heating elements 6 can be arranged in the area of the free wick end 29 next to it or, if necessary, at least partially surrounding it. In particular, the heating resistor with the PTC areal heating elements 6 can be arranged on a ring part, for example a ceramic ring part known per se, through which the free wick end 29 is inserted during operation. In the upper region of the housing component 4 above the free wick end 29, an outlet opening 24 is arranged for the substance to be discharged into the environment.

### List of reference signs

- 1: Electrical, flat heating resistor
- 2: Plug component
- 3: Container
- 4: Housing component
- 5a, b: Element rows
- 6a, b, c, d, e, f,: PTC areal heating elements
- 7: Edge length
- 8: Intermediate distance
- 9: Base carrier layer
- 10: Film layer conductor grid
- 11a, b: First and second main line
- 12a, b: Plug-in elements
- 13: Electrical connection element
- 14a, b: Stub lines from first main line
- 15a, b: Stub lines from second main line
- 16: Cover plate
- 17: Row spacing
- 18: Plug-in contacts
- 19: Capillary element
- 20: Flat wick
- 21a, b: U-leg
- 22: Substance dispensing area
- 23: Cover
- 24: Outlet opening
- 25: Heat conductive plate
- 26: Cover element
- 27: Delivery opening
- 28: Wick
- 29: Free wick end
- 30: Stub line distance

## Claims

1. Device for dispensing volatile substances,
- with a container (3) for the substance to be dispensed,
- with at least one capillary element (19) which is arranged at least partially in the container (3) and which is in contact with the substance to be dispensed and conveys it by means of capillary action to at least one substance dispensing area (22),
- with an electrical heating device with at least one electrical heating resistor (1) arranged in the region of the capillary element (19),
- with an electrical connection element (13) by means of which the electrical heating device is suppliable with electrical energy,
**characterized in that**
- the heating resistor (1) is formed by at least two element rows (5) spaced apart from one another, each element row (5) having at least one areal heating element (6), ,
- the areal heating elements (6) are applied as film layer resistors to an electrically non-conductive base carrier layer (9),
- an electrically conductive film layer conductor grid (10) is applied to the base carrier layer (9) for supplying electrical energy to the areal heating elements (6).

2. Device according to claim 1, **characterized in that,**
- a first main line (11a) and a second main line (11b) of the film layer conductor grid (10) are arranged on opposite sides of the at least two rows of elements (5) and are connectable to poles of different voltage of the electrical connection element (13),
- starting from the first main line (11a), a respective stub line (14) runs along each row of elements (5) and makes electrical contact with the at least one areal heating element (6) of each row of elements (5),
- starting from the second, opposite main line (11b), a respective stub line (15) runs along each row of elements (5) and makes electrical contact with the at least one areal heating element (6) of each row of elements (5), and
- the stub lines (14) of the first main line (11a) and the stub lines (15) of the second main line (11b) each have a stub line distance (30) between associated areal heating elements (6) of an element row (5).

3. Device according to claim 1 or 2, **characterized in that** at least two areal heating elements (6) spaced apart from one another by intermediate distances (8) are arranged in an element row (5) and/or **in that** an equal number of areal heating elements (6) is provided in each element row (5).

4. Device according to any one of the preceding claims, **characterized in that** the stub lines (14, 15) originating from the first main line (11a) and from the second main line (11b) each run with an electrical contact through an interconnection below or above the areal heating elements (6) of each row of elements (5).

5. Device according to any one of the preceding claims, **characterized in that** the areal heating elements (6) forming film layer resistors are formed by an electrically conductive ink or paint having a resistance function, which are applied to the base carrier layer (9) as a film layer, preferably printed and dried.

6. Device according to any one of the preceding claims, **characterized in that** the electrically conductive film layer conductor grid (10) is formed by an electrically conductive ink or paint having a current-carrying function, preferably comprising silver, which is applied to the base carrier layer (9) as a film layer, preferably printed and dried.

7. Device according to any one of the preceding claims, **characterized in that,**
the base carrier layer (9) is formed by the container (3) or is a component of the container (3),
a container holder is provided to which the container (3) is detachably connected,
the container holder has the electrical connection element (13) to which the container (3) is electrically connectable.

8. Device according to claim 7, **characterized in that** an electrical contact device, preferably an electrical contact device with plug-in elements (12a, 12b), is provided on the main lines (11a, 11b), by means of which the two main lines (11a, 11b) for the electrical power supply of the areal heating elements (6) are directly or indirectly connectable to the electrical connection element (13).

9. Device according to claim 7 or 8, **characterized in that,**
the container holder is formed as a plug component (2), and
the electrical connection element (13) has projecting plug contacts (18) which are pluggable into an electrical socket for supplying electrical power to the areal heating elements (6) and for holding the plug component (2) and the container (3) connected to the plug component (2).

10. Device according to any one of the claims 7 to 9, **characterized in that** the base carrier layer (9) is directly an outer side of a container wall part or a non-conductive intermediate layer connected to a container wall part, wherein in a heat transfer region a capillary element region to be heated adjoins the base carrier layer (9) with the areal heating elements (6), eventually with the interposition of an areal heat transfer element (25).

11. Device according to claim 10, **characterized in that** the capillary element (19) is a U-shaped wick (20), which is in contact with the substance to be dispensed with both U-legs (21a, 21b) in the container (3), the U-base part forming the substance dispensing area (22).

12. Device according to any one of claims 1 to 6, **characterized in that,**
a container holder is provided to which the container (3) is detachably connected,
the container holder is formed by a housing component (4) in which the container (3) is at least partially accommodated,
the base carrier layer (9) with the areal heating elements (6) is part of the housing component (4) and is arranged in the interior of the housing component (4) in the region of the inserted container (3), and
the housing component (4) has the electrical connection element (13) to which the container (3) is electrically connected.

13. Device according to claim 12, **characterized in that** the base carrier layer (9) with the areal heating elements (6) is arranged in the region of a substance dispensing area of a capillary element to be heated, most preferably connected to a free wick end (29) of a wick (28) forming a capillary element, the free wick end (29) protruding from the container (3).

14. Device according to claim 12 or 13, **characterized in that,**
the housing component (4) is designed as a connector component, and
the electrical connection element (13) has projecting plug contacts (18) which are pluggable into an electrical socket for supplying electrical power to the areal heating elements (6) and for holding the plug component (2) and the container (3) connected to the plug component (2).

15. Method of making a device for dispensing volatile substances,
- with a container (3) for the substance to be dispensed,
- with at least one capillary element (19) which is arranged at least partially in the container (3) and which is in contact with the substance to be dispensed and conveys it by means of capillary action to at least one substance dispensing area (22),
- with an electrical heating device with at least one electrical heating resistor (1) arranged in the region of the capillary element (19),
- with an electrical connection element (13) by means of which the electrical heating device can be supplied with electrical energy,
**characterized in that**
- the heating resistor (1) is produced with at least two element rows (5) spaced apart from one another, each element row (5) having at least one areal heating element (6),
- the areal heating elements (6) are applied as film layer resistors to an electrically non-conductive base carrier layer (9),
- an electrically conductive film layer conductor grid (10) is applied to the base carrier layer (9) for supplying electrical energy to the areal heating elements (6).

## Patentansprüche

1. Vorrichtung zur Abgabe flüchtiger Substanzen,
- mit einem Behälter (3) für die abzugebende Substanz,
- mit wenigstens einem Kapillarelement (19), das wenigstens teilweise in dem Behälter (3) angeordnet ist und das mit der abzugebenden Substanz in Kontakt steht und diese mittels Kapillarwirkung zu wenigstens einem Substanzabgabebereich (22) fördert,
- mit einer elektrischen Heizvorrichtung mit wenigstens einem elektrischen Heizwiderstand (1), der im Bereich des Kapillarelements (19) angeordnet ist,
- mit einem elektrischen Anschlusselement (13), mittels dessen die elektrische Heizvorrichtung mit elektrischer Energie versorgbar ist,
**dadurch gekennzeichnet, dass**
- der Heizwiderstand (1) durch wenigstens zwei voneinander beabstandete Elementreihen (5) gebildet ist, wobei jede Elementreihe (5) wenigstens ein Flächenheizelement (6) aufweist,
- die Flächenheizelemente (6) als Filmschichtwiderstände auf eine elektrisch nichtleitende Basisträgerschicht (9) aufgebracht sind,
- ein elektrisch leitfähiges Filmschicht-Leitergitter (10) auf die Basisträgerschicht (9) zum Zuführen elektrischer Energie zu den Flächenheizelementen (6) aufgebracht ist.

2. Vorrichtung nach Anspruch 1**, dadurch gekennzeichnet, dass**
- eine erste Hauptleitung (11a) und eine zweite Hauptleitung (11b) des Filmschicht-Leitergitters (10) auf gegenüberliegenden Seiten der wenigstens zwei Elementreihen (5) angeordnet und mit Polen unterschiedlicher Spannung des elektrischen Anschlusselements (13) verbindbar sind,
- ausgehend von der ersten Hauptleitung (11a) jeweils eine Stichleitung (14) entlang jeder Elementreihe (5) verläuft und das wenigstens eine Flächenheizelement (6) jeder Elementreihe (5) elektrisch kontaktiert,
- ausgehend von der zweiten, gegenüberliegenden Hauptleitung (11b) jeweils eine Stichleitung (15) entlang jeder Elementreihe (5) verläuft und das wenigstens eine Flächenheizelement (6) jeder Elementreihe (5) elektrisch kontaktiert, und
- die Stichleitungen (14) der ersten Hauptleitung (11a) und die Stichleitungen (15) der zweiten Hauptleitung (11b) jeweils einen Stichleitungsabstand (30) zwischen zugeordneten Flächenheizelementen (6) einer Elementreihe (5) aufweisen.

3. Vorrichtung nach Anspruch 1 oder 2**, dadurch gekennzeichnet, dass** wenigstens zwei voneinander durch Zwischenabstände (8) beabstandete Flächenheizelemente (6) in einer Elementreihe (5) angeordnet sind und/oder dass in jeder Elementreihe (5) eine gleiche Anzahl von Flächenheizelementen (6) vorgesehen ist.

4. Vorrichtung nach einem der vorhergehenden Ansprüche, **dadurch gekennzeichnet, dass** die von der ersten Hauptleitung (11a) und von der zweiten Hauptleitung (11b) ausgehenden Stichleitungen (14, 15) jeweils mit einem elektrischen Kontakt durch eine Verschaltung unterhalb oder oberhalb der Flächenheizelemente (6) jeder Elementreihe (5) verlaufen.

5. Vorrichtung nach einem der vorhergehenden Ansprüche, **dadurch gekennzeichnet, dass** die Filmschichtwiderstände bildenden Flächenheizelemente (6) durch eine elektrisch leitfähige Tinte oder Farbe mit einer Widerstandsfunktion gebildet sind, die als eine Filmschicht auf die Basisträgerschicht (9) aufgebracht sind, vorzugsweise gedruckt und getrocknet.

6. Vorrichtung nach einem der vorhergehenden Ansprüche, **dadurch gekennzeichnet, dass** das elektrisch leitfähige Filmschicht-Leitergitter (10) durch eine elektrisch leitfähige Tinte oder Farbe mit einer stromführenden Funktion, vorzugsweise Silber umfassend, gebildet ist, die als eine Filmschicht auf die Basisträgerschicht (9) aufgebracht ist, vorzugsweise gedruckt und getrocknet.

7. Vorrichtung nach einem der vorhergehenden Ansprüche, **dadurch gekennzeichnet, dass**
die Basisträgerschicht (9) durch den Behälter (3) gebildet ist oder ein Bestandteil des Behälters (3) ist,
ein Behälterhalter vorgesehen ist, mit dem der Behälter (3) lösbar verbunden ist,
der Behälterhalter das elektrische Anschlusselement (13) aufweist, mit dem der Behälter (3) elektrisch verbindbar ist.

8. Vorrichtung nach Anspruch 7**, dadurch gekennzeichnet, dass** an den Hauptleitungen (11a, 11b) eine elektrische Kontaktvorrichtung, vorzugsweise eine elektrische Kontaktvorrichtung mit Steckelementen (12a, 12b), vorgesehen ist, mittels der die beiden Hauptleitungen (11a, 11b) für die elektrische Energieversorgung der Flächenheizelemente (6) direkt oder indirekt mit dem elektrischen Anschlusselement (13) verbindbar sind.

9. Vorrichtung nach Anspruch 7 oder 8**, dadurch gekennzeichnet, dass**
der Behälterhalter als ein Steckerteil (2) ausgebildet ist, und
das elektrische Anschlusselement (13) vorstehende Steckkontakte (18) aufweist, die zur Versorgung der Flächenheizelemente (6) mit elektrischer Energie und zum Halten des Steckerteils (2) und des mit dem Steckerteil (2) verbundenen Behälters (3) in eine elektrische Steckdose einsteckbar sind.

10. Vorrichtung nach einem der Ansprüche 7 bis 9**, dadurch gekennzeichnet, dass** die Basisträgerschicht (9) direkt eine Außenseite eines Behälterwandteils oder eine mit einem Behälterwandteil verbundene nichtleitende Zwischenschicht ist, wobei in einem Wärmeübertragungsbereich ein zu erwärmender Kapillarelementbereich an die Basisträgerschicht (9) mit den Flächenheizelementen (6) angrenzt, gegebenenfalls unter Zwischenanordnung eines flächigen Wärmeübertragungselements (25).

11. Vorrichtung nach Anspruch 10**, dadurch gekennzeichnet, dass** das Kapillarelement (19) ein U-förmiger Docht (20) ist, der mit beiden U-Schenkeln (21a, 21b) in dem Behälter (3) mit der abzugebenden Substanz in Kontakt steht, wobei der U-Basisteil den Substanzabgabebereich (22) bildet.

12. Vorrichtung nach einem der Ansprüche 1 bis 6, **dadurch gekennzeichnet, dass**
ein Behälterhalter vorgesehen ist, mit dem der Behälter (3) lösbar verbunden ist,
der Behälterhalter durch ein Gehäusebauteil (4) gebildet ist, in dem der Behälter (3) wenigstens teilweise aufgenommen ist,
die Basisträgerschicht (9) mit den Flächenheizelementen (6) Teil des Gehäusebauteils (4) ist und im Inneren des Gehäusebauteils (4) im Bereich des eingesetzten Behälters (3) angeordnet ist, und
das Gehäusebauteil (4) das elektrische Anschlusselement (13) aufweist, mit dem der Behälter (3) elektrisch verbunden ist.

13. Vorrichtung nach Anspruch 12, **dadurch gekennzeichnet, dass** die Basisträgerschicht (9) mit den Flächenheizelementen (6) im Bereich eines Substanzabgabebereichs eines zu erwärmenden Kapillarelements angeordnet ist, höchst bevorzugt mit einem freien Dochtende (29) eines ein Kapillarelement bildenden Dochts (28) verbunden, wobei das freie Dochtende (29) aus dem Behälter (3) herausragt.

14. Vorrichtung nach Anspruch 12 oder 13, **dadurch gekennzeichnet, dass**
das Gehäusebauteil (4) als ein Anschlussbauteil ausgebildet ist, und
das elektrische Anschlusselement (13) vorstehende Steckkontakte (18) aufweist, die zur Versorgung der Flächenheizelemente (6) mit elektrischer Energie und zum Halten des Steckerteils (2) und des mit dem Steckerteil (2) verbundenen Behälters (3) in eine elektrische Steckdose einsteckbar sind.

15. Verfahren zur Herstellung einer Vorrichtung zur Abgabe flüchtiger Substanzen,
- mit einem Behälter (3) für die abzugebende Substanz,
- mit wenigstens einem Kapillarelement (19), das wenigstens teilweise in dem Behälter (3) angeordnet ist und das mit der abzugebenden Substanz in Kontakt steht und diese mittels Kapillarwirkung zu wenigstens einem Substanzabgabebereich (22) fördert,
- mit einer elektrischen Heizvorrichtung mit wenigstens einem elektrischen Heizwiderstand (1), der im Bereich des Kapillarelements (19) angeordnet ist,
- mit einem elektrischen Anschlusselement (13), mittels dessen die elektrische Heizvorrichtung mit elektrischer Energie versorgt werden kann,
**dadurch gekennzeichnet, dass**
- der Heizwiderstand (1) mit wenigstens zwei voneinander beabstandeten Elementreihen (5) hergestellt wird, wobei jede Elementreihe (5) wenigstens ein Flächenheizelement (6) aufweist,
- die Flächenheizelemente (6) als Filmschichtwiderstände auf eine elektrisch nichtleitende Basisträgerschicht (9) aufgebracht werden,
- ein elektrisch leitfähiges Filmschicht-Leitergitter (10) auf die Basisträgerschicht (9) zum Zuführen elektrischer Energie zu den Flächenheizelementen (6) aufgebracht wird.

## Revendications

1. Dispositif pour la distribution de substances volatiles,
- avec un récipient (3) pour la substance à distribuer,
- avec au moins un élément capillaire (19) qui est disposé au moins partiellement dans le récipient (3) et qui est en contact avec la substance à distribuer et l'achemine au moyen d'une action capillaire vers au moins une zone de distribution de substance (22),
- avec un dispositif de chauffage électrique comprenant au moins une résistance chauffante électrique (1) disposée dans la région de l'élément capillaire (19),
- avec un élément de connexion électrique (13) au moyen duquel le dispositif de chauffage électrique peut être alimenté en énergie électrique,
**caractérisé en ce que**
- la résistance chauffante (1) est formée par au moins deux rangées d'éléments (5) espacées les unes des autres, chaque rangée d'éléments (5) comprenant au moins un élément chauffant surfacique (6),
- les éléments chauffants surfaciques (6) sont appliqués sous forme de résistances en couche pelliculaire sur une couche support de base électriquement non conductrice (9),
- une grille conductrice en couche pelliculaire électriquement conductrice (10) est appliquée sur la couche support de base (9) pour fournir de l'énergie électrique aux éléments chauffants surfaciques (6).

2. Dispositif selon la revendication 1, **caractérisé en ce que**
- une première ligne principale (11a) et une deuxième ligne principale (11b) de la grille conductrice en couche pelliculaire (10) sont disposées sur des côtés opposés des au moins deux rangées d'éléments (5) et sont connectables à des pôles de tension différente de l'élément de connexion électrique (13),
- à partir de la première ligne principale (11a), une ligne de dérivation respective (14) s'étend le long de chaque rangée d'éléments (5) et établit un contact électrique avec l'au moins un élément chauffant surfacique (6) de chaque rangée d'éléments (5),
- à partir de la deuxième ligne principale opposée (11b), une ligne de dérivation respective (15) s'étend le long de chaque rangée d'éléments (5) et établit un contact électrique avec l'au moins un élément chauffant surfacique (6) de chaque rangée d'éléments (5), et
- les lignes de dérivation (14) de la première ligne principale (11a) et les lignes de dérivation (15) de la deuxième ligne principale (11b) présentent chacune un écartement des lignes de dérivation (30) entre des éléments chauffants surfaciques associés (6) d'une rangée d'éléments (5).

3. Dispositif selon la revendication 1 ou 2, **caractérisé en ce qu'**au moins deux éléments chauffants surfaciques (6) espacés les uns des autres par des distances intermédiaires (8) sont disposés dans une rangée d'éléments (5) et/ou **en ce qu'**un nombre égal d'éléments chauffants surfaciques (6) est prévu dans chaque rangée d'éléments (5).

4. Dispositif selon l'une quelconque des revendications précédentes, **caractérisé en ce que** les lignes de dérivation (14, 15) partant de la première ligne principale (11a) et de la deuxième ligne principale (11b) s'étendent chacune avec un contact électrique par une interconnexion au-dessous ou au-dessus des éléments chauffants surfaciques (6) de chaque rangée d'éléments (5).

5. Dispositif selon l'une quelconque des revendications précédentes, **caractérisé en ce que** les éléments chauffants surfaciques (6) formant des résistances en couche pelliculaire sont formés par une encre ou une peinture électriquement conductrice ayant une fonction de résistance, lesquels sont appliqués sur la couche support de base (9) sous forme d'une couche pelliculaire, de préférence imprimés et séchés.

6. Dispositif selon l'une quelconque des revendications précédentes, **caractérisé en ce que** la grille conductrice en couche pelliculaire électriquement conductrice (10) est formée par une encre ou une peinture électriquement conductrice ayant une fonction de conduction du courant, comprenant de préférence de l'argent, qui est appliquée sur la couche support de base (9) sous forme d'une couche pelliculaire, de préférence imprimée et séchée.

7. Dispositif selon l'une quelconque des revendications précédentes, **caractérisé en ce que**
la couche support de base (9) est formée par le récipient (3) ou est un composant du récipient (3),
un support de récipient est prévu auquel le récipient (3) est relié de manière amovible,
le support de récipient comporte l'élément de connexion électrique (13) auquel le récipient (3) peut être connecté électriquement.

8. Dispositif selon la revendication 7, **caractérisé en ce qu'**un dispositif de contact électrique, de préférence un dispositif de contact électrique avec des éléments enfichables (12a, 12b), est prévu sur les lignes principales (11a, 11b), au moyen duquel les deux lignes principales (11a, 11b) pour l'alimentation électrique des éléments chauffants surfaciques (6) sont directement ou indirectement connectables à l'élément de connexion électrique (13).

9. Dispositif selon la revendication 7 ou 8, **caractérisé en ce que**
le support de récipient est formé comme un composant enfichable (2), et
l'élément de connexion électrique (13) présente des contacts enfichables saillants (18) qui sont enfichables dans une prise électrique pour fournir de l'énergie électrique aux éléments chauffants surfaciques (6) et pour maintenir le composant enfichable (2) et le récipient (3) relié au composant enfichable (2).

10. Dispositif selon l'une quelconque des revendications 7 à 9, **caractérisé en ce que** la couche support de base (9) est directement un côté extérieur d'une partie de paroi du récipient ou une couche intermédiaire non conductrice reliée à une partie de paroi du récipient, dans lequel, dans une région de transfert thermique, une région d'élément capillaire à chauffer est adjacente à la couche support de base (9) avec les éléments chauffants surfaciques (6), éventuellement avec interposition d'un élément surfacique de transfert thermique (25).

11. Dispositif selon la revendication 10, **caractérisé en ce que** l'élément capillaire (19) est une mèche en forme de U (20), qui est en contact avec la substance à distribuer avec les deux branches en U (21a, 21b) dans le récipient (3), la partie de base en U formant la zone de distribution de substance (22).

12. Dispositif selon l'une quelconque des revendications 1 à 6, **caractérisé en ce que**
un support de récipient est prévu auquel le récipient (3) est relié de manière amovible,
le support de récipient est formé par un composant de boîtier (4) dans lequel le récipient (3) est au moins partiellement reçu,
la couche support de base (9) avec les éléments chauffants surfaciques (6) fait partie du composant de boîtier (4) et est disposée à l'intérieur du composant de boîtier (4) dans la région du récipient inséré (3), et
le composant de boîtier (4) comporte l'élément de connexion électrique (13) auquel le récipient (3) est connecté électriquement.

13. Dispositif selon la revendication 12, **caractérisé en ce que** la couche support de base (9) avec les éléments chauffants surfaciques (6) est disposée dans la région d'une zone de distribution de substance d'un élément capillaire à chauffer, de manière particulièrement préférée reliée à une extrémité libre de mèche (29) d'une mèche (28) formant un élément capillaire, l'extrémité libre de mèche (29) faisant saillie hors du récipient (3).

14. Dispositif selon la revendication 12 ou 13, **caractérisé en ce que** le composant de boîtier (4) est conçu comme un composant connecteur, et l'élément de connexion électrique (13) présente des contacts enfichables saillants (18) qui sont enfichables dans une prise électrique pour fournir de l'énergie électrique aux éléments chauffants surfaciques (6) et pour maintenir le composant enfichable (2) et le récipient (3) relié au composant enfichable (2).

15. Procédé de fabrication d'un dispositif pour la distribution de substances volatiles,
- avec un récipient (3) pour la substance à distribuer,
- avec au moins un élément capillaire (19) qui est disposé au moins partiellement dans le récipient (3) et qui est en contact avec la substance à distribuer et l'achemine au moyen d'une action capillaire vers au moins une zone de distribution de substance (22),
- avec un dispositif de chauffage électrique comprenant au moins une résistance chauffante électrique (1) disposée dans la région de l'élément capillaire (19),
- avec un élément de connexion électrique (13) au moyen duquel le dispositif de chauffage électrique peut être alimenté en énergie électrique,
**caractérisé en ce que**
- la résistance chauffante (1) est produite avec au moins deux rangées d'éléments (5) espacées les unes des autres, chaque rangée d'éléments (5) comprenant au moins un élément chauffant surfacique (6),
- les éléments chauffants surfaciques (6) sont appliqués sous forme de résistances en couche pelliculaire sur une couche support de base électriquement non conductrice (9),
- une grille conductrice en couche pelliculaire électriquement conductrice (10) est appliquée sur la couche support de base (9) pour fournir de l'énergie électrique aux éléments chauffants surfaciques (6).
